# EUROPEAN PATENT APPLICATION

(11) **EP 2 570 088 A1**
(43) Date of publication of application: **20.03.2013**
(21) Application number: 12006512.3
(22) Date of filing: 17.09.2012
(51) Int. Cl.: A61B 17/16

(54) **Endoscopic ronguer with vacuum or impulsive sytem for removing broken-off bone parts**

(30) Priority: 19.09.2011 US 201113235564
(71) Applicant: Aygun, Mehmet, 55070 Samsun Turkey (TR)
(72) Inventor: Aygun, Mehmet, 55070 Samsun Turkey (TR)
(74) Representative: Yavan, Nuriye

(57) **Abstract**

The invention relates to a bone cutter that is designed for surgical purposes, that can be used with vacuum or Impulsive system depending on the preference of the surgeon and that is manufactured in two different, disposable or reusable models, cutter edges with various diameter and size can be installed on the same leg mechanism.

## Description

The invention relates to a bone cutter with a vacuum or impulsive system consisting of both a disposable or reusable models that have edges that can be fixed in any desired direction. Such a bone cutter is designed for surgical applications, and can rotate around its axis, which cutter edges with various diameters and length can be installed on a single leg mechanism.

A punch that prevents incorrect operation through its short impact and that can be easily removed from the operation area is described in the patent document JP 2008178696 (A).

A surgical device that is used to reach a part within the body, which includes a shaft and a squeezable punch to take out parts adjacent to the first end of that shaft, is described in patent document US 5643283 (A).

A punch used to implant or remove an organ, organ parts, or a tissue complex into or out of a human or animal body is described in patent document DE 4405831 (A1).

A medical device, which embodies an inner organ or tissue during operations, and especially during endoscopic operations, is described in patent document US 5341815 (A).

There are only reusable models in osteotomic punch systems in the state of the art. If there is any fault in the surgical device, it is not possible to resolve the problem through replacing the defective part or parts by the operator. In such a case, the defective surgical device has to either be sent to the manufacturer or the technical service, or scraped.

Not all parts of the known osteotomic punches can be disassembled for cleaning and sterilization purposes. If a surgeon disassembles any parts of the device, he/she has to match the serial numbers of the parts in order to reassemble the device. This causes some difficulties in assembling the parts. Such difficulties have an inhibitory and risk-increasing structure in carrying out sufficient cleaning and sterilization of the device. Costs of such devices are high due to their fixed structures.

Through a disposable model of the bone cutter invention with a vacuum or impulsive system, the aforementioned requirement of the existing punches for disassembly and reassembly has been removed. Most of the parts of the disposable bone cutter have been manufactured from plastic material.

Parts of the reusable model of the bone cutter invention with vacuum or impulsive system have been manufactured from metal. In such models, the operator may be required to disassemble and replace the defective part among the components of the surgical device. However, no wrench or screwdriver or any other assisting tool is required to *perform such a process.* All parts can be disassembled into its components or assembled manually by performing pushing and turning movements in the required direction. The help of such features ensures cleaning and sterilization safety. Furthermore, service waiting time and scraping of the device, along with its all components, are avoided. The device has a structure that allows spare part management.

Another disadvantage of existing bone cutters is that for each part the surgeon breaks off, it is imperative to remove the device out of the operation area, empty the cutter edge, and return to the operation area. In such a case, the risk of the operation increases, and the duration of the operation and anesthesia are affected adversely.

### SUMMARY OF THE INVENTION

A bone cutter invention with vacuum or impulsive system provides two different solutions for such problems of the existing models. Such solutions are provided through an impulsive shaft mechanism or vacuum apparatus. When the bone cutter is used with the impulsive shaft mechanism, depending on the preference of the surgeon, the broken-off parts are removed from the operation area by means of the impulsive mechanism. When the device is used with a vacuum apparatus installed instead of the impulsive shaft mechanism, broken-off parts and liquid are vacuumed through the groove within the shaft. In both situations, the surgical device is used without removing it from the operation area after each cutting process. Thus, risk of the operation is reduced, and operation and anesthesia time is saved.

### BRIEF DESCRIPTION OF THE DRAWINGS

The subject of the invention, the bone cutter system invention with vacuum or impulsive system will be better understood from the below drawings, in which:

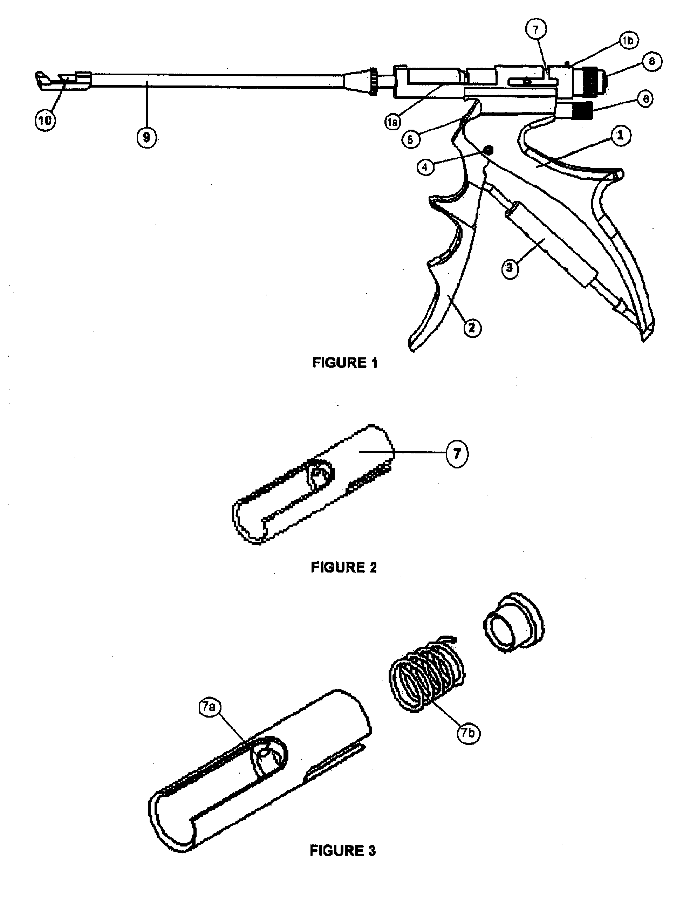

Figure 1 is the drawing of the bone cutter with vacuum or impulsive system.

Figure 2 is the drawing of the cutter edge fixing mechanism.

Figure 3 is the drawing of the disassembled view of the cutter edge fixing mechanism.

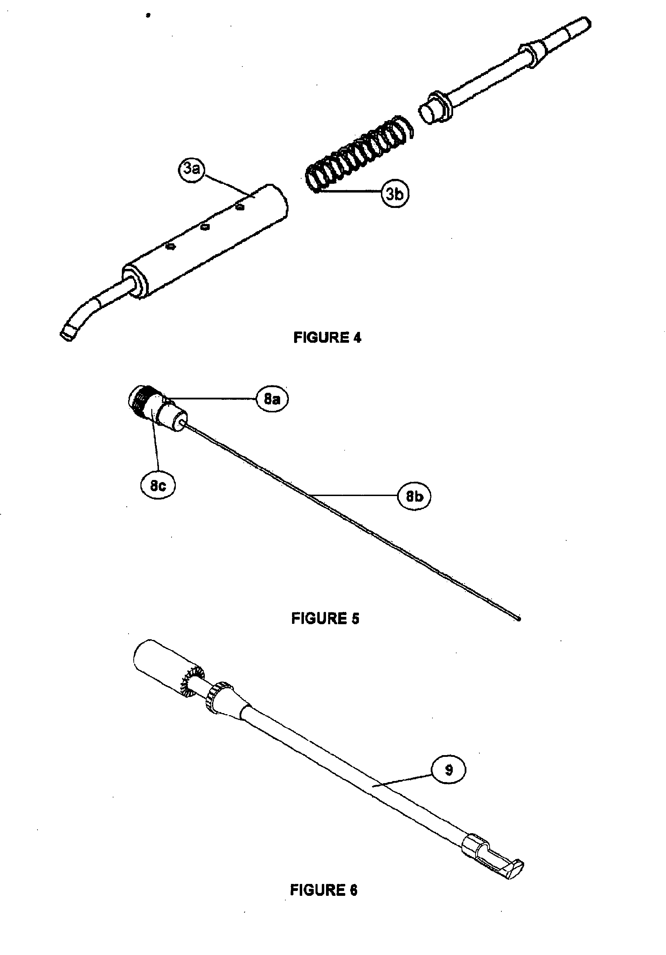

Figure 4 is the drawing of the disassembled view of the parts of the grip spring piston.

Figure 5 is the drawing of the impulsive shaft mechanism.

Figure 6 is the drawing of the cutter edge outer tube.

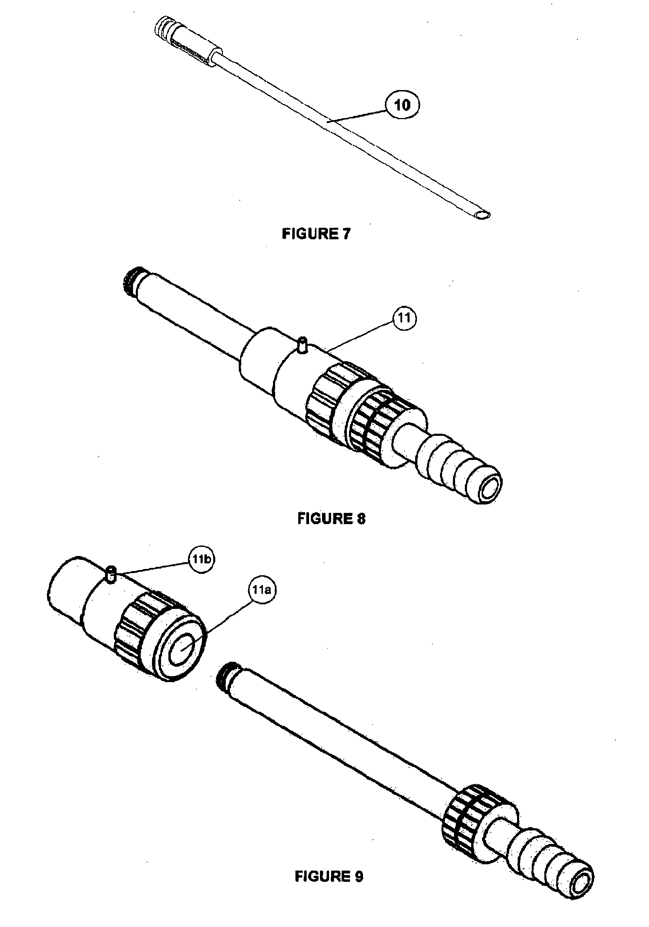

Figure 7 is the drawing of the cutter edge inner tube.

Figure 8 is the drawing of the vacuum apparatus.

Figure 9 is the drawing of the disassembled view of the vacuum apparatus.

### DRAWING REFERENCE NUMBERS

1. Grip fixed leg
   1.a. grip fixed leg front groove
   1.b. grip fixed leg seat
2. Grip moving leg
3. Grip spring piston
   3.a. Grip spring piston outer tube
   3.b. Grip spring piston spring
4. Grip moving leg fixing pin
5. Locking joint pin
6. Operating distance adjustment mechanism
7. Cutter edge fixing mechanism
   7.a. Cutter edge fixing mechanism handle
   7.b. Cutter edge fixing mechanism spring
8. Impulsive shaft mechanism
   8.a. Locking lug
   8.b. Impulsive spindle
   8.c. Shaft
9. Cutter edge outer tube
10. Cutter edge inner tube
11. Vacuum apparatus
   11.a. Vacuum apparatus fixing seat
   11.b.Vacuum apparatus locking lug

### DESCRIPTION OF THE EMBODIMENTS

The bone cutter with vacuum or impulsive system has been developed to be used with the impulsive shaft mechanism (8) or vacuum apparatus (11), depending on the desire of the operator. A surgeon can use the system through mounting the apparatus he/she needs to the system in the system he desires. The bone cutter has been manufactured in a disposable or reusable manner according to the needs of the user. Most of the parts of the disposable cutter have been manufactured from plastic material, while all parts of the reusable cutter have been manufactured from metal material.

In the first preferred embodiment of the bone cutter invention with vacuum or impulsive system, the cutter edge inner tube (10) is placed in the cutter edge outer tube (9). The end of the cutter edge inner tube (10) plays a cutting role. The combined such two parts are then placed in the groove (1a) on the front portion of the grip fixed leg (1). After such process, the cutter edge fixing mechanism (7) is brought forward and by this way the cutter edge inner tube (10) and the cutter edge outer tube (9) are fixed. Afterwards, the impulsive shaft mechanism (8) is placed in the groove (1a) in the front portion of the grip fixed leg (1) in such a way that it is in the same axis as the cutter edge inner tube (10) and the cutter edge fixing mechanism (7). Then the locking lug (8a), existing on the impulsive shaft mechanism, (8) is mounted through placing it in the seat (1b) on the grip fixed leg (1).

For the breaking off process, the grip moving leg (2) is squeezed with the palm of the hand and so the cutter edge inner tube (10) closes, providing the breaking off process. When the grip moving leg (2) is released, the grip spring piston (3) acts, and the grip moving leg (2) returns to its previous position through movement of the spring (3b) that is placed within the grip piston outer tube (3a). In such a case, the cutter edge inner tube (10) re-opens. This way, the broken-off part is removed through the mouth section by means of the impulsive spindle (8b) within the shaft (8c).

The operating distance adjustment mechanism (6) is used to adjust the operating distance of the cutter edge. It is mounted on the grip fixed leg (1) by means of threads opened on it in a screw-like shape and it adjusts the movement distance of the grip moving leg (2) at the point touching to the grip moving leg (2) and therefore the operating distance of the cutter edge inner tube (10), which moves in conjunction with the grip moving leg, is adjusted.

The grip moving leg fixing pin (4) is the mechanism connected to the body, which continuously holds it at a forward position by means of the spring within it, by threads opened on it in a screw-like shape. By this way, the main parts of the system and the grip moving leg (2) are fixed with each other. The locking joint pin (5) is a pin designed in a screw-less L shape used to combine the grip fixed leg (1) and the grip moving leg (2) parts. By this way, it is aimed to ensure coordinated movement of the grip fixed leg (1) and the grip moving leg (2) and fix them with each other.

The cutter edge fixing mechanism (7) is a tube-shaped part that contains two grooves. It contains one handle (7a) for the holding process and one spring (7b) for the impulsion mechanism process.

In the second preferred embodiment of the bone cutter invention with a vacuum or impulsive system, when the operator desires to work through aspiration, the impulsive shaft mechanism (8) is removed and the vacuum apparatus (11) is installed instead. Similar to the impulsive shaft mechanism (8), it is placed in the groove (1a) in the front portion of the grip fixed leg (1) in such a way that it is on the same axis as the cutter edge inner tube (10) and the cutter edge fixing mechanism (7). Then the locking lug (11a) existing on the vacuum apparatus (11) is mounted by placing it in the seat (1b) on the grip fixed leg (1). Then the vacuum apparatus-fixing seat (11a) is placed in the fixed leg front groove (1a) in order to fix it to the moving area. Then the vacuuming process is performed through connecting it to the vacuum device with a hose.

## Claims

1. A bone cutter used for surgical purposes, comprising: a grip fixed leg (1); a grip moving leg (2); a grip spring piston (3); a grip moving leg fixing pin (4) that is attached to the bone cutter's main body; a locking joint pin (5) that connects the grip fixed leg (1) and the grip moving leg (2); an operating distance adjustment mechanism (6) that is mounted on the grip fixed leg (1); a cutter edge fixing mechanism (7); a cutter edge outer tube (9) that is placed in the grip fixed leg (1); and a cutter edge inner tube (10) that is placed in the cutter edge outer tube (9); and an impulsive shaft mechanism (8);
wherein all of the bone cutter can be manually assembled and disassembled;

2. The bone cutter as claimed in claim 1 wherein the bone cutter is disposable and the material used is plastic.

3. The bone cutter as claimed in claim 1 wherein the bone cutter is reusable and the material used is metal.

4. The bone cutter as claimed in claim 2 further comprising an impulsive shaft mechanism (8).

5. The bone cutter as claimed in claim 3 further comprising an impulsive shaft mechanism (8).

6. The bone cutter as claimed in claim 2 further comprising a vacuum apparatus (11).

7. The bone cutter as claimed in claim 3 further comprising a vacuum apparatus (11).

8. A bone cutter according to claim 1, further comprising: a groove (1.a.) in the front portion of the grip fixed leg (1) in which the combined the cutter edge inner tube (10) and the cutter edge outer tube (9) are placed.

9. A bone cutter according to claim 4 wherein the grip fixed leg (1) contains a seat (1.b.) on which a locking lug (8.a.) on the impulsive shaft mechanism (8) is installed.

10. A bone cutter according to claim 5 wherein the grip fixed leg (1) contains a seat (1.b.) on which a locking lug (8.a.) on the impulsive shaft mechanism (8) is installed.

11. A bone cutter according to claim 1, wherein when the grip moving leg (2) is released, a grip spring piston outer tube (3.a.) returns the grip moving leg (2) to its previous position.

12. A bone cutter according to claim 1, wherein the grip spring piston spring (3.b.) placed within the grip spring piston outer tube (3.a).

13. A bone cutter according to claim 1, wherein the operating distance adjustment mechanism (6) is connected on the grip fixed leg (1) by means of plurality of threads opened on it in a screw shape and it adjusts the movement distance of the grip fixed leg (1) and the cutter edge inner tube (10) moving in conjunction with it, at the point touching to the grip moving leg (2).

14. A bone cutter according to the claim 1, further comprising: a handle (7.a.) for holding process that is placed on the cutter edge fixing mechanism (7).

15. A bone cutter according to the claim 1 wherein the cutter edge fixing mechanism (7), which has a tube shape with two grooves on it, contains a spring (7.b.) for impulsion process.

16. The bone cutter according to claim 1, wherein when the grip spring piston (3) acts, the cutter edge inner tube (10) re-opens and the broken-off part is removed through the mouth section by means of the impulsive spindle (8.b.) within the shaft (8.c).

17. The bone cutter according to claim 6, wherein the vacuum apparatus (11) comprises a fixing seat (11.a.) on it allowing easy assembly of the apparatus on the cutter.

18. The bone cutter according to claim 6, wherein the vacuum apparatus (11) comprises a locking lug (11.b) on it

19. The bone cutter according to claim 7, wherein the vacuum apparatus (11) comprises a fixing seat (11.a.) on it allowing easy assembly of the apparatus on the cutter.

20. The bone cutter according to claim 7, wherein the vacuum apparatus (11) comprises a locking lug (11.b) on it.
